# FASCICULE DE BREVET EUROPEEN

(11) **EP 1 090 627 B1**
(45) Date de publication et mention de la délivrance du brevet: **27.03.2002**
(21) Numéro de dépôt: 00402325.5
(22) Date de dépôt: 21.08.2000
(51) Int. Cl.: A61K 7/48, A61K 7/06, A61K 7/02

(54) **Composition sous forme d'émulsion huile-dans-eau contenant des fibres, et ses utilisations notamment cosmétiques**
Faserhaltige Zusammensetzung vom Typ Öl-in-Wasser Emulsion und ihre Verwendung in der Kosmetik
Oil-in-water emulsion composition containing fibers and use of said composition in cosmetics

(30) Priorité: 07.10.1999 FR 9912504
(43) Date de publication de la demande: 11.04.2001
(73) Titulaire: L'OREAL, 75008 Paris (FR)
(72) Inventeur: Afriat, Isabelle, 75003 Paris (FR)
(74) Mandataire: Rasson, Catherine

(56) Documents cités:
- EP-A- 0 268 164
- EP-A- 0 838 210
- PATENT ABSTRACTS OF JAPAN vol. 1999, no. 01, 29 janvier 1999 (1999-01-29) & JP 10 287523 A (POLA CHEM IND INC), 27 octobre 1998 (1998-10-27)

## Description

La présente demande se rapporte à une composition sous forme d'émulsion huile-dans-eau contenant des fibres et un copolymère acrylique particulier, et à l'utilisation de la dite composition, en particulier pour le soin, le traitement et/ou le maquillage de la peau du corps ou du visage, des cheveux, des cils et/ou des lèvres, et pour le soin des peaux sensibles.

Il est connu par le document JP07-196440 des compositions cosmétiques contenant des fibres de polyamide courtes, celles-ci donnant aux dites compositions un toucher velouté et une bonne tenue cosmétique. Toutefois, l'incorporation de ces fibres de polyamide dans les émulsions huile dans eau (H/E) pose des problèmes de stabilité, c'est-à-dire que les émulsions déphasent à température ambiante ou à des températures plus élevées, et ce notamment quand la quantité de fibres est importante.

Il subsiste donc le besoin d'émulsions H/E contenant des fibres, et notamment des fibres de polyamide, et présentant de bonnes propriétés cosmétiques sans avoir les inconvénients de l'art antérieur.

La demanderesse a découvert de façon inattendue que les polymères comportant dans leur chaîne une partie hydrophile et une partie hydrophobe constituée d'une chaîne grasse permettaient de réaliser des émulsions huile-dans-eau contenant des fibres, stables même en l'absence de tout tensioactif classiquement utilisé pour stabiliser les émulsions H/E.

La présente invention concerne une composition sous forme d'émulsion huile-dans-eau comprenant dans un milieu physiologiquement acceptable, une phase huileuse dispersée dans une phase aqueuse, caractérisée en ce qu'elle contient des fibres et au moins un copolymère, éventuellement réticulé, d'une fraction majoritaire de monomère acide carboxylique monooléfiniquement insaturé en C₃-C₆ ou de son anhydride, et d'une fraction minoritaire de monomère ester à chaîne grasse d'acide acrylique, les fibres n'étant pas des fibres de cellulose ayant une longueur de 1 à 40 µm.

On entend ici par « milieu physiologiquement acceptable », un milieu compatible avec la peau, les lèvres, le cuir chevelu, les cils, les yeux et/ou les cheveux.

La composition obtenue a l'aspect d'une crème (produit souple par opposition à produit solide dur) et elle a une texture veloutée, agréable à l'application. En outre, même en l'absence de tensioactif classiquement utilisé pour les émulsions H/E, tel que les esters béhéniques polyoxyéthylénés ou les esters gras de sorbitan, elle reste stable dans le temps à température ambiante ou à des températures plus élevées. Ainsi selon un mode préféré de réalisation de l'invention, la composition de l'invention est exempte de tensioactif classique. Ainsi, du fait de l'absence de tensioactif, elle présente l'avantage de ne pas être irritante pour les peaux particulièrement sensibles et de permettre, en outre, l'incorporation d'actifs thermosensibles car elle peut être fabriquée à température ambiante.

Les fibres utilisables dans la composition de l'invention peuvent être des fibres hydrophiles ou hydrophobes, d'origine synthétique ou naturelle, minérale ou organique, à l'exclusion des fibres de cellulose ayant une longueur de 1 à 40 µm. Elles peuvent être courtes ou longues, unitaires ou organisées par exemple tressées. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 nm à 20 mm, de préférence de 10 nm à 5 mm et mieux de 0,1 mm à 1,5 mm. Leur section peut être comprise dans un cercle de diamètre allant de 2 nm à 100 µm, de préférence allant de 20 nm à 20 µm et mieux de 5 µm à 20 µm. Le poids des fibres est souvent donné en denier ou décitex.

Les fibres peuvent être celles utilisées dans la fabrication des textiles et notamment des fibres de soie, de coton, de laine, de lin, de cellulose extraites notamment du bois, des légumes ou des algues, de polyamide (Nylon® ), de rayonne, de viscose, d'acétate notamment d'acétate de rayonne, de poly-p-phénylène téréphtamide notamment de Kevlar® , en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon® , de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et de caprolactone (Monocryl de la société Johnson & Johnson) ; les fibres synthétiques résorbables du type copolymère d'acide lactique et d'acide glycolique (Vicryl de la société Johnson & Johnson) ; les fibres de polyester téréphtalique (Ethibond de la société Johnson & Johnson) et les fils d'acier inoxydable (Acier de la société Johnson & Johnson).

Par ailleurs, les fibres peuvent être traitées ou non en surface, enrobées ou non. Comme fibres enrobées utilisables dans l'invention, on peut citer des fibres de polyamide enrobées de sulfure de cuivre pour un effet antistatique (par exemple R-STAT de la société Rhodia) ou un autre polymère permettant une organisation particulière des fibres (traitement de surface spécifique) ou un traitement de surface induisant des effets de couleurs/hologrammes (fibre Lurex de la société Sildorex, par exemple).

Les fibres utilisables dans la composition selon l'invention sont de préférence des fibres de polyamide et/ou de poly-p-phénylènetéréphtamide. Leur longueur peut aller de 0,1 à 5 mm, de préférence de 0,25 à 1,6 mm, et leur diamètre moyen peut aller de 5 à 50 µm. En particulier, on peut utiliser les fibres de polyamide commercialisées par les Etablissements P. Bonte sous le nom Polyamide 0,9 Dtex 0,3 mm, ayant un diamètre moyen de 6 µm, un poids d'environ 0,9 dtex et une longueur allant de 0,3 mm à 1,5 mm. On peut aussi utiliser les fibres de poly-p-phénylène téréphtamide de diamètre moyen de 12 pm et de longueur d'environ 1,5 mm comme celles vendues sous le nom de Kevlar Floc par la société Du Pont Fibres. Selon un mode particulier de réalisation de l'invention, ces fibres sont introduites dans la phase huileuse de l'émulsion.

Les fibres peuvent être présentes dans la composition selon l'invention en une quantité allant de 0,1 à 20 % en poids et de préférence de 0,5 à 15 % en poids par rapport au poids total de la composition.

Le copolymère constitué d'une fraction majoritaire de monomère acide carboxylique monooléfiniquement insaturé en C₃-C₆ ou de son anhydride, et d'une fraction minoritaire de monomère ester à chaîne grasse d'acide acrylique, utilisable dans la composition de l'invention, peut être préparé en polymérisant une quantité prépondérante de monomère carboxylique monooléfiniquement insaturé ou de son anhydride, avec une quantité plus faible de monomère ester acrylique à chaîne grasse. La quantité de monomère carboxylique ou de son anhydride, va de préférence de 80 à 98 % en poids et plus particulièrement de 90 à 98 % en poids ; l'ester acrylique est de préférence présent dans des quantités allant de 2 à 20 % en poids et plus particulièrement de 2 à 10 % en poids; les pourcentages sont calculés par rapport au poids des deux monomères.

Les monomères carboxyliques préférentiels sont choisis parmi ceux répondant à la formule :

CH₂=CR-COOH

dans laquelle R désigne l'hydrogène, un halogène, le groupe hydroxyle, un groupe lactone, un groupe lactame, un groupe cyanogène (-CN), un groupe alkyle monovalent, un groupe aryle, un groupe alkylaryle, un groupe aralkyle ou un groupe cycloaliphatique. De préférence, R désigne l'hydrogène ou un groupe alkyle comportant de 1 à 6 atomes de carbone, notamment les radicaux méthyle et éthyle.

Les monomères carboxyliques particulièrement préférés sont choisis parmi l'acide acrylique, l'acide méthacrylique, l'anhydride maléique, et leurs mélanges.

Les monomères esters acryliques à chaîne grasse sont préférentiellement choisis parmi ceux répondant à la formule :

CH₂=CR¹-COOR²

dans laquelle R¹ est choisi dans le groupe formé par l'hydrogène, le radical méthyle et le radical éthyle, et R² est un groupe alkyle en C₈-C₃₀, un groupe oxyalkylène en C₈-C₃₀, un groupe carbonyloxyalkylène en C₈-C₃₀.

Les monomères esters particulièrement préférés sont ceux pour lesquels R¹ est l'hydrogène ou le radical méthyle, et/ou ceux pour lesquels R² est un groupe alkyle en C₁₀-C₂₂. On peut notamment citer les acrylates et méthacrylates de décyle, de lauryle, de stéaryle, de béhényle ou de mélissyle.

Certains des copolymères selon l'invention sont notamment décrits dans le document EP-A-0268164 et sont obtenus selon les méthodes de préparation décrites dans ce document.

On peut citer plus particulièrement les copolymères vendus sous le nom PEMULEN par la Société GOODRICH, et notamment le copolymère acrylate/C₁₀-C₃₀-alkylacrylate tel que le produit PEMULEN TR1 ou le PEMULEN TR2.

On peut, bien évidemment, utiliser un mélange de plusieurs copolymères tels que ci-dessus définis.

Ces copolymères peuvent être présents dans les compositions selon l'invention en une quantité allant de 0,01 à 3 % en poids par rapport au poids total de la composition, de préférence 0,02 à 0,6 % en poids, et plus préférentiellement de 0,05 à 0,2 % en poids.

La phase huileuse de la composition selon l'invention représente généralement de 10 à 50 % et de préférence de 15 à 30 % en poids par rapport au poids total de la composition.

La phase huileuse peut être constituée par tous les corps gras et notamment les huiles, classiquement utilisés dans les domaines cosmétique ou dermatologique.

Parmi les huiles utilisables dans l'émulsion de l'invention, on peut citer par exemple les huiles végétales telles que les huiles de jojoba, avocat, amande douce, abricot, maïs et la fraction liquide de beurre de karité ; les huiles minérales comme l'huile de vaseline et le polyisobutène hydrogéné ; les huiles de synthèse comme le palmitate d'éthyl-2 hexyle, le myristate d'isopropyle, l'isoparaffine hydrogénée, l'isononanoate d'isononyle, l'octanoate de cétéaryle ; les huiles de silicone volatiles (cyclométhicones par exemple) ou non volatiles (polydiméthylsiloxanes ou PDMS) et les huiles fluorées. Les autres corps gras susceptibles d'être présents dans la phase huileuse peuvent être par exemple les acides gras et les alcools gras.

La phase aqueuse de la composition de l'invention constitue en général de 30 à 85 % et de préférence de 60 à 75 % en poids par rapport au poids total de la composition.

De façon connue, les compositions de l'invention peuvent contenir des adjuvants habituels dans les domaines considérés, tels que des actifs hydrophiles ou lipophiles, des conservateurs, des gélifiants, des antioxydants, des parfums, des solvants, des charges ou des nacres, des filtres, des matières colorantes (pigments ou colorants solubles), des agents basiques ou acides et encore des vésicules lipidiques. Ces adjuvants sont utilisés dans les proportions habituelles dans le domaine cosmétique, et par exemple de 0,01 à 30 % du poids total de l'émulsion, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour l'émulsion de l'invention.

Comme actifs, on peut citer par exemple les hydratants tels que les polyols comme la glycérine et le sorbitol ; les agents kératolytiques ; les dépigmentants ; les amincissants, et tout actif approprié pour le but final de la composition.

Selon la fluidité de la composition que l'on souhaite obtenir, on peut y ajouter un ou plusieurs gélifiants hydrophiles ou lipophiles. Comme gélifiants hydrophiles, on peut citer par exemple les polymères carboxyvinyliques, tels que les carbomers. Comme gélifiants lipophiles, on peut citer les argiles modifiées telles que les bentones telles que le mélange « cyclomethicone, Quaternium-18 hectorite, SD alcohol 40 » (10/85/5) (nom CTFA) commercialisé sous la dénomination Bentone Gel VS-5 par la société Rheox ; les organopolysiloxanes élastomères réticulés tels que ceux commercialisés sous les noms KSG6 de Shin-Etsu, Trefil E-505C ou Trefil E-506C de Dow-Corning, Gransil de Grant Industries (SR-CYC, SR DMF10, SR-DC556), ou ceux commercialisés sous forme de gels : KSG15, KSG17, KSG16, KSG18 de Shin-Etsu, Gransil SR 5CYC gel, Gransil SR DMF 10 gel, Gransil SR DC 556 gel de Grant Industries, SF 1204 et JK 113 de General Electric.

Ces gélifiants, lorsqu'ils sont présents, sont généralement utilisés à des concentrations allant de 0,1 à 7 % et de préférence de 0,1 à 5 % en poids de matière active par rapport au poids total de la composition.

Les compositions, objets de l'invention, trouvent leur application dans un grand nombre de traitements notamment cosmétiques et peuvent ainsi constituer une composition cosmétique, notamment pour le traitement, la protection, le soin, le démaquillage et/ou le nettoyage de la peau, des lèvres et/ou des cheveux, et/ou pour le maquillage de la peau, des lèvres, des cils et/ou du corps.

Les compositions selon l'invention peuvent par exemple être utilisées comme produits de soin, de démaquillage et/ou de nettoyage pour le visage sous forme de crèmes ou de laits ou comme produits de maquillage (peau, cils et lèvres) par incorporation de pigments ou de colorants, par exemple comme fonds de teint.

Aussi, l'invention a pour objet l'utilisation cosmétique de la composition telle que définie ci-dessus pour le traitement, la protection, le soin, le démaquillage et/ou le nettoyage de la peau, des lèvres et/ou des cheveux, et/ou pour le maquillage de la peau, des lèvres, des cils et/ou du corps.

L'invention a aussi pour objet un procédé de traitement cosmétique de la peau, y compris du cuir chevelu, des cheveux, des cils et/ou des lèvres, caractérisé par le fait qu'on applique sur la peau, les cheveux, les cils et/ou les lèvres, une composition telle que définie ci-dessus.

Du fait que la composition peut être exempte de tensioactif classique, elle est particulièrement bien tolérée par les sujets ayant une peau sensible.

L'invention a donc encore pour objet l'utilisation de la composition telle que définie ci-dessus pour la fabrication d'une composition destinée au soin des peaux sensibles.

Les exemples qui suivent permettront de mieux comprendre l'invention. Les quantités indiquées sont en % en poids, sauf mention contraire.

| **Exemple 1 : Crème protectrice de jour** | |
|---|---|
| *Phase huileuse :* | |
| Cyclométhicone (cyclopentasiloxane) | 12 % |
| KSG 16 (à 24 % de matière active) | 4 % |
| Bentone Gel VS-5 (commercialisé par la société Rheox) | 2 % |

| *Phase aqueuse :* | |
|---|---|
| Carbomer | 0,3 % |
| Pemulen TR2 | 0,3 % |
| Triéthanolamine | 0,6 % |
| Conservateurs | 1 % |
| Eau déminéralisée | qsp 100 % |
| Fibres de polyamide | |
| (Polyamide 0,9 Dtex, 0,3 mm - Société Paul Bonte) | 12 % |

Mode opératoire : on prépare la phase aqueuse en ajoutant à l'eau les conservateurs et le carbomer qui doit être bien dispersé. Puis, on ajoute le Pemulen qui doit aussi être bien dispersé. Par ailleurs, on prépare l'empâtage des fibres en les mélangeant à la phase huileuse. Puis on passe ce mélange à la tricylindre de façon à obtenir un mélange le plus homogène possible. On émulsionne l'empâtage en le versant petit à petit dans la phase aqueuse sous forte agitation. Enfin, on ajoute la triéthanolamine.

On obtient une crème qui reste stable dans le temps, même après conservation à 45°C. A l'application sur la peau, elle est d'une grande douceur et est particulièrement adapté pour les peaux grasses.

| **Exemple 2 : Crème de jour** | |
|---|---|
| *Phase huileuse :* | |
| Cyclométhicone (cyclopentasiloxane) | 18 % |

| *Phase aqueuse :* | |
|---|---|
| Carbomer | 0,3 % |
| Pemulen TR2 | 0,3 % |
| Triéthanolamine | 0,6 % |
| Conservateurs | 1 % |
| Eau déminéralisée | qsp 100 % |
| Fibres de polyamide (Polyamide 0,9 Dtex, 0,3 mm - Société Paul Bonte) | 8 % |

Mode opératoire: on prépare la phase aqueuse en ajoutant à l'eau les conservateurs et le carbomer qui doit être bien dispersé. Puis, on ajoute le Pemulen qui doit aussi être bien dispersé. Par ailleurs, on disperse les fibres dans la phase huileuse. On émulsionne le mélange en le versant petit à petit dans la phase aqueuse sous forte agitation. Enfin, on ajoute la triéthanolamine.

On obtient une crème qui reste stable dans le temps, même après conservation à 45°C. A l'application sur la peau, elle est d'une grande légèreté et est particulièrement adaptée pour les peaux couperosées.

## Revendications

1. Composition sous forme d'émulsion huile-dans-eau comprenant dans un milieu physiologiquement acceptable, une phase huileuse dispersée dans une phase aqueuse, **caractérisée en ce qu'**elle contient des fibres et au moins un copolymère, éventuellement réticulé, d'une fraction majoritaire de monomère acide carboxylique monooléfiniquement insaturé en C₃-C₆ ou de son anhydride, et d'une fraction minoritaire de monomère ester à chaîne grasse d'acide acrylique, les fibres n'étant pas des fibres de cellulose ayant une longueur de 1 à 40 µm.

2. Composition selon la revendication 1, **caractérisée en ce que** les fibres ont une longueur allant de 0,1 à 5 mm.

3. Composition selon la revendication 1 ou 2, **caractérisée en ce que** les fibres ont une section comprise dans un cercle de diamètre allant de 5 à 50 µm.

4. Composition selon l'une quelconque des revendications précédentes, **caractérisée en ce que** les fibres sont choisies parmi les fibres de polyamide, les fibres de poly-p-phénylènetéréphtamide et leurs mélanges.

5. Composition selon l'une quelconque des revendications précédentes, **caractérisée en ce que** les fibres sont présentes en une quantité allant de 0,1 % à 15 % en poids par rapport au poids total de la composition.

6. Composition selon l'une quelconque des revendications précédentes, **caractérisée en ce que** le copolymère est un copolymère acrylate/C₁₀-C₃₀-alkylacrylate.

7. Composition selon l'une quelconque des revendications précédentes, **caractérisée en ce que** le copolymère est présent en une quantité allant de 0,01 à 3 % en poids par rapport au poids total de la composition.

8. Composition selon l'une quelconque des revendications précédentes, **caractérisée en ce que** la phase huileuse représente de 10 à 50 % en poids par rapport au poids total de la composition.

9. Composition selon l'une quelconque des revendications précédentes, **caractérisée par le fait qu'**elle comprend au moins un gélifiant.

10. Composition selon la revendication précédente, **caractérisée en ce que** le gélifiant est choisi parmi les polymères carboxyvinylique, les organopolysiloxanes élastomères réticulés, les argiles modifiées et leurs mélanges.

11. Composition selon l'une quelconque des revendications précédentes, **caractérisée en ce qu'**elle constitue une composition cosmétique.

12. Utilisation cosmétique de la composition selon l'une quelconque des revendications 1 à 11, pour le traitement, la protection, le soin, le démaquillage et/ou le nettoyage de la peau, des lèvres et/ou des cheveux, et/ou pour le maquillage de la peau, des lèvres, des cils et/ou du corps.

13. Procédé de traitement cosmétique de la peau, des cheveux, des cils et/ou des lèvres, **caractérisé par le fait qu'**on applique sur la peau, les cheveux, les cils et/ou les lèvres, une composition selon l'une quelconque des revendications 1 à 11.

14. Utilisation de la composition selon l'une quelconque des revendications 1 à 11, pour la fabrication d'une composition destinée au soin des peaux sensibles.

## Patentansprüche

1. Zusammensetzung, die in Form einer Öl-in-Wasser-Emulsion vorliegt und die in einem physiologisch akzeptablen Medium eine in einer Ölphase dispergierte wäßrige Phase enthält, **dadurch gekennzeichnet, daß** sie Fasern und mindestens ein gegebenenfalls vernetztes Copolymer enthält, das hauptsächlich aus monoolefinisch ungesättigten C₃₋₆-Carbonsäuremonomeren oder ihren Anhydriden und zu einem geringeren Anteil aus Acrylsäureestermonomeren mit Fettkette besteht, wobei es sich bei den Fasern nicht um Cellulosefasern mit einer Länge von 1 bis 40 µm handelt.

2. Zusammensetzung nach Anspruch 1, **dadurch gekennzeichnet, daß** die Fasern eine Länge von 0,1 bis 5 mm aufweisen.

3. Zusammensetzung nach Anspruch 1 oder 2, **dadurch gekennzeichnet, daß** die Fasern einen Querschnitt aufweisen, der in einen Kreis mit einem Durchmesser von 5 bis 50 µm einbeschrieben werden kann.

4. Zusammensetzung nach einem der vorhergehenden Ansprüche, **dadurch gekennzeichnet, daß** die Fasern unter den Polyamidfasern oder den Fasern aus Poly-p-phenylenterephthamid oder deren Gemischen ausgewählt sind.

5. Zusammensetzung nach einem der vorhergehenden Ansprüche,
**dadurch gekennzeichnet, daß** die Fasern in einem Mengenanteil von 0,1 bis 15 Gew.-%, bezogen auf das Gesamtgewicht der Zusammensetzung, vorliegen.

6. Zusammensetzung nach einem der vorhergehenden Ansprüche,
**dadurch gekennzeichnet, daß** das Copolymer ein Acrylat/C₁₀₋₃₀-Alkylacrylat ist.

7. Zusammensetzung nach einem der vorhergehenden Ansprüche, **dadurch gekennzeichnet, daß** das Copolymer in einem Mengenanteil von 0,01 bis 3 Gew.-%, bezogen auf das Gesamtgewicht der Zusammensetzung, vorliegt.

8. Zusammensetzung nach einem der vorhergehenden Ansprüche, **dadurch gekennzeichnet, daß** die Ölphase 10 bis 50 Gew.-%, bezogen auf das Gesamtgewicht der Zusammensetzung, ausmacht.

9. Zusammensetzung nach einem der vorhergehenden Ansprüche, **dadurch gekennzeichnet, daß** sie mindestens einen Gelbildner enthält.

10. Zusammensetzung nach dem vorhergehenden Anspruch, **dadurch gekennzeichnet, daß** der Gelbildner unter den Carboxyvinylpolymeren, vernetzten elastomeren Organopolysiloxanen, modifizierten Tonen und deren Gemischen ausgewählt ist.

11. Zusammensetzung nach einem der vorhergehenden Ansprüche, **dadurch gekennzeichnet, daß** es sich um eine kosmetische Zusammensetzung handelt.

12. Kosmetische Verwendung der Zusammensetzung nach einem der Ansprüche 1 bis 11 zur Behandlung, zum Schutz, zur Pflege, zum Abschminken und/oder zur Reinigung der Haut, der Lippen und/oder der Haare und/oder zum Schminken der Haut, der Lippen, der Wimpern und/oder des Körpers.

13. Verfahren zur kosmetischen Behandlung der Haut, der Haare, der Wimpern und/oder der Lippen, **dadurch gekennzeichnet, daß** auf die Haut, die Haare, die Wimpern und/oder die Lippen eine Zusammensetzung nach einem der Ansprüche 1 bis 11 aufgetragen wird.

14. Verwendung der Zusammensetzung nach einem der Ansprüche 1 bis 11 zur Herstellung einer Zusammensetzung, die zur Pflege empfindlicher Haut vorgesehen ist.

## Claims

1. Composition in the form of an oil-in-water emulsion comprising, in a physiologically acceptable medium, an oily phase dispersed in an aqueous phase, **characterized in that** it comprises fibres and at least one optionally crosslinked copolymer with a major fraction of monoolefinically unsaturated C₃-C₆ carboxylic acid monomer or monomer of its anhydride and with a minor fraction of acrylic acid fattychain ester monomer, the fibres not being cellulose fibres having a length of 1 to 40 µm.

2. Composition according to Claim 1, **characterized in that** the fibres have a length ranging from 0.1 to 5 mm.

3. Composition according to Claim 1 or 2, **characterized in that** the fibres have a cross section included within a circle with a diameter ranging from 5 to 50 µm.

4. Composition according to any one of the preceding claims, **characterized in that** the fibres are chosen from polyamide fibres, poly(p-phenylene terephthalamide) fibres and their mixtures.

5. Composition according to any one of the preceding claims, **characterized in that** the fibres are present in an amount ranging from 0.1% to 15% by weight with respect to the total weight of the composition.

6. Composition according to any one of the preceding claims, **characterized in that** the copolymer is an acrylate/C₁₀-C₃₀ alkyl acrylate copolymer.

7. Composition according to any one of the preceding claims, **characterized in that** the copolymer is present in an amount ranging from 0.01 to 3% by weight with respect to the total weight of the composition.

8. Composition according to any one of the preceding claims, **characterized in that** the oily phase represents from 10 to 50% by weight with respect to the total weight of the composition.

9. Composition according to any one of the preceding claims, **characterized in that** it comprises at least one gelling agent.

10. Composition according to the preceding claim, **characterized in that** the gelling agent is chosen from carboxyvinyl polymers, crosslinked elastomeric organopolysiloxanes, modified clays and their mixtures,

11. Composition according to any one of the preceding claims, **characterized in that** it constitutes a cosmetic composition.

12. Cosmetic use of the composition according to any one of Claims 1 to 11 for treating, protecting, caring for, removing make-up from and/or cleansing the skin, lips and/or hair and/or for making up the skin, lips, eyelashes and/or body.

13. Process for the cosmetic treatment of the skin, hair, eyelashes and/or lips, **characterized in that** a composition according to any one of Claims 1 to 11 is applied to the skin, hair, eyelashes and/or lips.

14. Use of the composition according to any one of Claims 1 to 11 in the manufacture of a composition intended for caring for sensitive skin.
